# EUROPÄISCHE PATENTSCHRIFT

(11) **EP 2 809 640 B1**
(45) Veröffentlichungstag und Bekanntmachung des Hinweises auf die Patenterteilung: **13.01.2016**
(21) Anmeldenummer: 13700460.2
(22) Anmeldetag: 15.01.2013
(51) Int. Cl.: C07C 41/01, C07C 43/04, C01B 3/34, C01B 31/18, C07C 1/20, C07C 11/04, C07C 11/06, C07C 1/22

(54) **VERFAHREN ZUR HERSTELLUNG VON DIMETHYLETHER AUS METHAN**
PROCESS FOR PREPARING DIMETHYL ETHER FROM METHANE
PROCÉDÉ DE PRÉPARATION D'ÉTHER DIMÉTHYLIQUE A PARTIR DE MÉTHANE

(30) Priorität: 31.01.2012 DE 102012001803; 31.01.2012 DE 102012001811; 21.02.2012 EP 12001135
(43) Veröffentlichungstag der Anmeldung: 10.12.2014
(73) Patentinhaber: Linde AG, 80331 München (DE)
(72) Erfinder: SCHÖDEL, Nicole, 81477 München (DE); HAIDEGGER, Ernst, 85521 Riemerling (DE); SCHMIGALLE, Holger, 82538 Geretsried (DE); BEHRENS, Axel, 80689 München (DE); GÖKE, Volker, 82538 Geretsried (DE); THALLER, Christian, 80687 München (DE); SCHMADERER, Harald, 82515 Wolfratshausen (DE)
(86) Internationale Anmeldenummer: PCT/EP2013/000102
(87) Internationale Veröffentlichungsnummer: WO 2013/113468

(56) Entgegenhaltungen:
- JP-A- 2000 103 757
- US-A1- 2006 287 405
- US-A1- 2008 319 093

## Beschreibung

Die Erfindung betrifft ein Verfahren zur Herstellung von Dimethylether aus Methan.

Ein solches Verfahren umfasst einen Trockenreformierungsschritt, wobei Methan und Kohlendioxid zu Kohlenmonoxid und Wasserstoff umgesetzt werden, und einen Syntheseschritt, wobei Kohlenmonoxid und Wasserstoff zu Dimethylether und Kohlendioxid umgesetzt werden.

Aus der US 2006/287405A1 ist ein Verfahren zur Herstellung von Dimethylether aus Kohlenwasserstoffen bekannt, wobei mittels Tri-Reformierung eines Ausgangsgemisches aus Kohlenwasserstoffen, Kohlendioxid und Wasserdampf in Gegenwart eines Tri-Reformierungskatalysators ein Synthesegas gebildet wird, das dann zu Dimethylether umgesetzt wird.

Weiterhin ist aus der JP2000 103757 A ein Verfahren zur Herstellung von DME ausgehend von niederen Kohlenwasserstoffen, Wasserdampf und Kohlendioxid bekannt, wobei zunächst Synthesegas hergestellt wird, das dann zu DME umgesetzt wird.

Schließlich ist aus der US 2008/319093 A1 ein Verfahren zur Herstellung von DME ausgehend von Methan und Kohlendioxid bekannt, wobei zunächst in einem kombinierten Trockenreformierungs- und Dampfreformierungsschritt aus Methan, Kohlendioxid und Wasserdampf ein Synthesegas hergestellt wird und dieses zu Methanol umgesetzt wird, dass dann zu DME dehydriert wird.

Dimethylether (DME) kann auf konventionellem Weg über die Dehydratisierung von Methanol (MeOH) hergestellt werden:

2 MeOH -7 DME + H₂O.

Methanol wird dabei typischerweise aus Synthesegas (H₂+CO) mit einem stöchiometrischen Verhältnis von 2 mol H₂ und 1 mol CO hergestellt:

2H₂ + CO → MeOH.

Im Gegensatz hierzu gibt es neuere Direkt-Verfahren, die aus einem Synthesegas mit einem stöchiometrischen H₂/CO-Verhältnis von etwa 1:1 in einem Schritt DME produzieren:

3 H₂ + 3 CO → DME + CO₂.

Bei der Erzeugung von Synthesegas aus Methan oder Erdgas auf konventionellem Weg, wie etwa durch autotherme Reformierung, Dampfreformierung oder partielle Oxidation, werden jedoch H₂/CO-Verhältnisse deutlich größer als 1:1 erzielt. Daher muss dieses Gas vor dem Einsatz in einer direkten DME-Synthese auf das richtige stöchiometrische H₂/CO-Verhältnis von ca. 1:1 gebracht und gegebenenfalls gereinigt werden.

Bei der Herstellung von Synthesegas durch Trockenreformierung ist eine aufwändige Kompression des Synthesegases vor der DME-Synthese notwendig.

Hiervon ausgehend liegt daher der vorliegenden Erfindung die Aufgabe zugrunde, ein apparativ einfaches und ökonomisch tragfähiges Verfahren zur Synthese von Dimethylether aus Methan zur Verfügung zu stellen.

Dieses Problem wird durch ein Verfahren mit den Merkmalen des unabhängigen Anspruchs 1 gelöst.

Danach ist vorgesehen, dass der Trockenreformierungsschritt und der Syntheseschritt bei Drücken durchgeführt werden, die sich nicht mehr als 3,0 bar, vorzugsweise nicht mehr als 1,0 bar voneinander unterscheiden. Vorzugsweise werden der Trockenreformierungsschritt und der Syntheseschritt bei im Wesentlichen gleichen Drücken durchgeführt.

Durch das Anpassen der Druckniveaus von Trockenreformierung und (direkter) DME-Synthese können apparativ aufwändige und kostenintensive Kompressionsstufen eingespart werden. Weiterhin kann prinzipiell durch die Trockenreformierung ein Gasgemisch bereitgestellt werden, welches ohne weitere Reinigung, Änderung, Kompression und/oder Expansion in der DME-Direktsynthese verwendet werden kann. Auch hierdurch können aufwändige Apparate mit Vorteil entfallen.

Die Trockenreformierung wird dabei vorteilhafterweise unter Anwesenheit eines modifizierten, rußresistenten nickelbasierten Katalysators durchgeführt, wie er auch bei Dampfreformierungen ähnlich zur Anwendung kommt. Zweckmäßigerweise wird die Trockenreformierung bevorzugt bei einer Temperatur zwischen 750°C und 950°C durchgeführt.

Methan im Sinne der Erfindung umfasst auch methanhaltige Gase wie Erdgas.

Unter Trockenreformierung im Sinne der Erfindung versteht man die Umsetzung von Methan oder Erdgas und CO₂ unter Wärmezufuhr und in Abwesenheit von Wasser zu Synthesegas mit einem stöchiometrischen Verhältnis von H₂ und CO von etwa 1:1. Die Trockenreformierung im Sinne der Erfindung umfasst auch die Umsetzung von CH₄ oder Erdgas und CO₂ in Anwesenheit von Wasserdampf, wobei Wasser nur in einem stöchiometrischen Verhältnis zu Methan oder Erdgas von 1:2, 1:3, 1:4, 1:5, 1:10 oder 1:20 anwesend ist. Generell wird im Rahmen dieser Erfindung von einer Trockenrefomierung gesprochen, wenn das molare Verhältnis von Wasser zu Kohlenstoff im Einsatz kleiner als 2:1, bevorzugt kleiner als 1:1, ist.

Unter dem Syntheseschritt im Sinne der Erfindung ist die direkte Dimethylethersynthese zu verstehen, bei der Dimethylether direkt aus Wasserstoff und Kohlenmonoxid entsteht.

Im Wesentlichen gleiche Drücke im Verständnis der Erfindung sind Drücke, die sich nicht mehr als 1,0 bar, bevorzugter noch 0,5 bar, 0,4 bar, 0,3 bar, 0,2 bar und am meisten bevorzugt nicht mehr als 0,1 bar voneinander unterscheiden. Die Drücke zwischen den beiden Syntheseschritten können z.B. durch den normalen Druckverlust der dazwischen benötigten Bauteile (z.B. Rohrleitungen) geringfügig voneinander abweichen. Dabei wird der Druck, der am Ausgang des Trockenreformierungsschritt vorliegt, mit dem Druck, der am Eingang in den DME-Syntheseschritt vorliegt, verglichen wird.

Die Trockenreformierung und/oder die direkte Dimethylethersynthese können in Anwesenheit geeigneter Katalysatoren, wie etwa Übergangsmetallkatalysatoren durchgeführt werden. Bei der Trockenreformierung sind insbesondere modifizierte, rußresistente, Ni-basierte Katalysatoren vorteilhaft, wie sie auch in anderen Verfahren der Dampfreformierung verwendet werden. Bei der Dimethylethersynthese werden vorteilhafterweise kupferbasierte Katalysatoren eingesetzt, die auch in anderen Verfahren der Methanolsynthese gebräuchlich sind.

Hierzu wird bevorzugt ein Cu-basierter Katalysator verwendet, der eine acide Funktionalität für eine entsprechende hohe Aktivität und Selektivität für DME aufweist (bifunktionaler Katalysator). Hierbei begünstigt jene Funktionalität insbesondere die Abspaltung von Wasser aus ggf. während der DME-Synthese auftretenden Methanol gemäß 2CH₃OH -> DME + H₂O.

Gemäß einer bevorzugten Ausführungsform der Erfindung wird das Verfahren bei einem Druck von 20 bar bis 50 bar durchgeführt. Die Erhöhung des Druckes gemäß dieser Ausgestaltung der Erfindung bietet verschiedene Vorteile. Bei der Dimethylethersynthese wird das Reaktionsgleichgewicht zu den Reaktionsprodukten verschoben. Bei der Trockenrefomierung wird zwar die Reaktionsausbeute leicht verringert, jedoch bieten sich durch den Wegfall der Kompressionstufe derartige verfahrenstechnische Vorteile, dass dies kompensiert wird.

Weiterhin ist bevorzugt keine Behandlung des Produktgases aus Trockenreformierung vor Eintritt in die DME-Synthese vorgesehen, da die Trockenreformierung bereits ein für die direkte DME-Synthese passendes Gas bereitstellt.

Gemäß einer weiter bevorzugten Ausführungsform der Erfindung wird der Syntheseschritt zumindest bis zu einem Zeitpunkt durchgeführt, bei dem Dimethylether in einer Konzentration von mindestens 60%, 70%, 80%, 90% oder 100% der Gleichgewichtskonzentration von Dimethylether vorliegt.

Die Gleichgewichtskonzentration von Dimethylether im Sinne der Erfindung bedeutet die Dimethyletherkonzentration, die vorliegt, wenn sich die Reaktion von Kohlenmonoxid und Wasserstoff zu Dimethylether und Kohlendioxid im chemischen Gleichgewicht befindet. Das chemische Gleichgewicht der Reaktion ist erreicht, wenn die Geschwindigkeit der Hinreaktion (3 H₂ + 3 CO → DME + CO₂) gleich der Geschwindigkeit der Rückreaktion (DME + CO₂ → 3 H₂ + 3 CO) ist.

Gemäß einer weiteren Ausführungsform der Erfindung wird das Produkt des Syntheseschritts in eine vorwiegend dimethylether-, methanol-, und wasserhaltige Produktphase und ein vorwiegend wasserstoff-, kohlenmonoxid-, kohlendioxid- und methanhaltiges Restgas getrennt.

Eine solche Trennung kann beispielsweise durch Abkühlung und Kondensation des im Syntheseschritt entstehenden Gases durchgeführt werden, wobei ein Kondensat vorwiegend bestehend aus Methanol, Wasser, Dimethylether und gelöstem Kohlendioxid und eine gasförmige Phase aus Wasserstoff, Kohlenmonoxid, Kohlendioxid und Methan entsteht. Nach Abtrennen des Kondensats von der gasförmigen Phase, kann Dimethylether vom Wasser und/oder Methanol durch Destillation, Rektifikation oder Sorption abgetrennt werden.

Eine Produktphase vorwiegend bestehend aus Methanol, Wasser und Dimethylether im Sinne der Erfindung bedeutet insbesondere, dass die Produktphase zu mindestens 60%, 70% oder 80% (Volumenprozent) aus Methanol, Wasser und/oder Dimethylether besteht. Ebenso bedeutet ein Restgas vorwiegend bestehend aus Wasserstoff, Kohlenmonoxid, Kohlendioxid und Methan insbesondere, dass das Restgas zu mindestens 60%, 70% oder 80% (Volumenprozent) aus Wasserstoff, Kohlenmonoxid, Kohlendioxid und/oder Methan besteht.

Gemäß einer weiteren Ausführungsform der Erfindung wird das vorwiegend wasserstoff-, kohlenmonoxid-, kohlendioxid- und methanhaltige Restgas dem Trockenreformierungsschritt wieder zugeführt. Diese Wiederverwertung des Restgases erhöht die Ausbeute des Verfahrens und reduziert die Menge der Abfallprodukte.

Gemäß einer alternativen Ausführungsform der Erfindung wird das wasserstoff-, kohlenmonoxid-, kohlendioxid- und methanhaltige Restgas zur Bereitstellung thermischer Energie für den endothermen Reformierungsschritt verwendet. Thermische Energie kann durch Oxidation der brennbaren Bestandteile des Restgases zu Wasser und Kohlendioxid erzeugt werden. Eine Zufuhr von thermischer Energie oder Wärme zum endothermen Reformierungsschritt kann das chemische Gleichgewicht der Reformierungsreaktion auf die Produktseite (Wasserstoff und Kohlenmonoxid) verschieben.

Weiterhin kann Dimethylether in einem Olefinsyntheseschritt zu einem Produkt umfassend Olefine, insbesondere Ethylen und/oder Propylen, umgesetzt werden, wobei Dimethylether, insbesondere nach alleiniger Abtrennung von CO₂, bzw. jene Produktphase direkt dem Olefinsyntheseschritt zugeführt wird.

Weitere Einzelheiten und Vorteile der Erfindung sollen durch die nachfolgenden Figurenbeschreibungen von Ausführungsbeispielen anhand der Figuren erläutert werden.

Es zeigt:

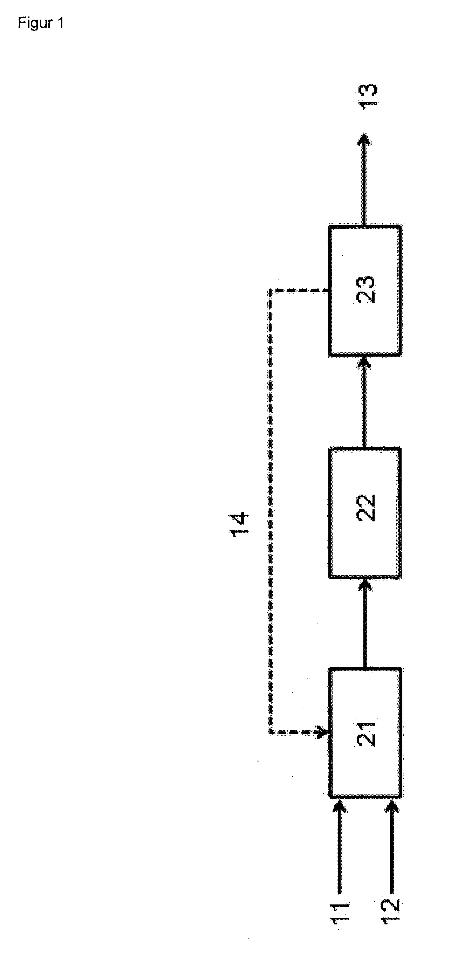

- Fig. 1: ein Schema des erfindungsgemäßen Verfahrens.

### Beispiel 1:

Das erfindungsgemäße Verfahren (Fig. 1) umfasst die Kombination von Trockenreformierung 21 zur Synthesegasherstellung mit direkter DME-Synthese 22 bei Durchführung der beiden Schritte auf gleichem Druckniveau in einem Druckbereich von 20 bar bis 50 bar, das heißt, ohne Kompression oder Entspannung zwischen dem Trockenreformierungsschritt 21 und dem DME-Syntheseschritt 22.

Das Produkt der Trockenreformierung 21 (Kohlenmonoxid und Wasserstoff) muss vor Eintritt in die DME-Synthese 22 keiner Behandlung wie Reinigung, Änderung der Verhältnisse von Kohlenmonoxid und Wasserstoff, Kompression oder Expansion unterzogen werden, da die Trockenreformierung 21 ein für die direkte DME-Synthese 22 passendes Gas bereitstellt. Durch das Anpassen der Druckniveaus von Trockenreformierung 21 und (direkter) DME-Synthese 22 wird die apparativ aufwändige und kostenintensive Kompressionsstufe eingespart.

Bevorzugt werden im Syntheseschritt 22 Wasserstoff und Kohlenmonoxid bis nahe der chemischen Gleichgewichtseinstellung der Reaktion zu Dimethylether und Kohlendioxid umgesetzt, so dass das im Syntheseschritt 22 entstehende Gas im Anschluss in eine vorwiegend DME/MeOH/H₂O-haltige Produktphase 13 und in ein vorwiegend H₂/CO/CO₂/CH₄-haltiges Restgas 14 getrennt werden kann (Fig. 1, 23). Das abgetrennte Restgas 14 muss nicht gereinigt und in der DME-Synthese 22 wiederverwandt werden, sondern kann in der Trockenreformierung 21 recycelt werden. Hierdurch ergibt sich eine verbesserte Integration, eine signifikante Verschlankung des Prozesses und damit geringere Investitionskosten.

Alternativ wird das Restgas 14 nicht stofflich in der Trockenreformierung 21 recycelt, sondern kann energetisch zur Beheizung der endothermen Synthesegasherstellung 21 verwendet werden.

**Bezugszeichenliste:**

| | |
|---|---|
| 11 | Methan oder Erdgas |
| 12 | Kohlendioxid |
| 13 | Produktphase (DME/Methanol/H₂O) |
| 14 | Restgas (H₂/CO/CO₂/CH₄) |
| 21 | Trockenreformierung |
| 22 | DME-Direktsynthese |
| 23 | Trennung Produktgas und Restgas |

## Patentansprüche

1. Verfahren zur Herstellung von Dimethylether aus Methan (11), umfassend:
- einen Trockenreformierungsschritt (21), wobei Methan (11) und Kohlendioxid (12) zu Kohlenmonoxid und Wasserstoff umgesetzt, und
- einen Syntheseschritt (22), wobei das im Trockenreformierungsschritt (21) entstandene Kohlenmonoxid und Wasserstoff zu Dimethylether (13) und Kohlendioxid umgesetzt wird,
**dadurch gekennzeichnet, dass**
der Trockenreformierungsschritt (21) und der Syntheseschritt (22) bei Drücken, die sich nicht mehr als 3,0 bar, vorzugsweise nicht mehr als 1,0 bar voneinander unterscheiden, durchgeführt werden.

2. Verfahren nach Anspruch 1, **dadurch gekennzeichnet, dass** das Verfahren bei einem Druck von 20 bar bis 50 bar durchgeführt wird.

3. Verfahren nach Anspruch 1 oder 2, **dadurch gekennzeichnet, dass** ein durch den Trockenreformierungsschritt (21) erzeugtes Produktgas enthaltend besagtes Kohlenmonoxid und besagten Wasserstoff direkt dem Syntheseschritt (22) zugeführt wird.

4. Verfahren nach einem der Ansprüche 1 bis 3, **dadurch gekennzeichnet, dass** beim Syntheseschritt (22) das im Trockenreformierungsschritt (21) entstandene Kohlenmonoxid und Wasserstoff zu Dimethylether (13) und Kohlendioxid bis zu einem Zeitpunkt umgesetzt wird, ab dem Dimethylether in einer Konzentration von mindestens 60%, 70%, 80%, 90 oder 100% der Gleichgewichtskonzentration von Dimethylether vorliegt.

5. Verfahren nach einem der Ansprüche 1 bis 4, **dadurch gekennzeichnet, dass** das Produkt des Syntheseschritts (22) in eine vorwiegend dimethylether-, methanol- und wasserhaltige Produktphase (13) und ein vorwiegend wasserstoff-, kohlenmonoxid-, kohlendioxid- und methanhaltiges Restgas (14) getrennt wird.

6. Verfahren nach Anspruch 5, **dadurch gekennzeichnet, dass** das vorwiegend wasserstoff-, kohlenmonoxid-, kohlendioxid- und methanhaltige Restgas (14) dem Trockenreformierungsschritt (21) zugeführt wird.

7. Verfahren nach Anspruch 5, **dadurch gekennzeichnet, dass** das vorwiegend wasserstoff-, kohlenmonoxid-, kohlendioxid- und methanhaltige Restgas (14) zur Bereitstellung von thermischer Energie für den Trockenreformierungsschritt (21) verwendet wird.

8. Verfahren nach einem der vorhergehenden Ansprüche, **dadurch gekennzeichnet, dass** Dimethylether (13) in einem Olefinsyntheseschritt zu einem Produkt umfassend Olefine, insbesondere Ethylen und/oder Propylen, umgesetzt wird, wobei Dimethylether (13), insbesondere nach alleiniger Abtrennung von CO₂, oder die Produktphase (13) direkt dem Olefinsyntheseschritt zugeführt wird.

## Claims

1. Process for production of dimethyl ether from methane (11) comprising:
- a dry-reforming step (21), wherein methane (11) and carbon dioxide (12) are converted into carbon monoxide and hydrogen, and
- a synthesis step (22), wherein the carbon monoxide and hydrogen formed in the dry-reforming step (21) are converted into a dimethyl ether (13) and carbon dioxide,
**characterized in that**
the dry-reforming step (21) and the synthesis step (22) are carried out at pressures which differ from each other by not more than 3.0 bar, preferably not more than 1.0 bar.

2. Process according to Claim 1, **characterized in that** the process is carried out at a pressure of 20 bar to 50 bar.

3. Process according to Claim 1 or 2, **characterized in that** a product gas generated by the dry-reforming step (21) and containing said carbon monoxide and said hydrogen is fed to the synthesis step (22) directly.

4. Process according to any of Claims 1 to 3, **characterized in that** the carbon monoxide and hydrogen formed in the dry-reforming step (21) is converted in the synthesis step (22) into dimethyl ether (13) and carbon dioxide until the dimethyl ether is present in a concentration amounting to not less than 60%, 70%, 80%, 90 or 100% of the equilibrium concentration of dimethyl ether.

5. Process according to any of Claims 1 to 4, **characterized in that** the product of synthesis step (22) is separated into a predominantly dimethyl ether-, methanol- and water-containing product phase (13) and a predominantly hydrogen-, carbon monoxide-, carbon dioxide- and methane-containing residual gas (14).

6. Process according to Claim 5, **characterized in that** the predominantly hydrogen-, carbon monoxide-, carbon dioxide- and methane-containing residual gas (14) is fed to the dry-reforming step (21).

7. Process according to Claim 5, **characterized in that** the predominantly hydrogen-, carbon monoxide-, carbon dioxide- and methane-containing residual gas (14) is used for providing thermal energy for the dry-reforming step (21).

8. Process according to any preceding claim, **characterized in that** dimethyl ether (13) is converted in an olefin synthesis step into a product comprising olefins, in particular ethylene and/or propylene, wherein the olefin synthesis step is fed with dimethyl ether (13) particularly after sole removal of CO₂ or with product phase (13) directly.

## Revendications

1. Procédé de fabrication d'éther diméthylique à partir de méthane (11), comprenant :
- une étape de reformage à sec (21), lors de laquelle du méthane (11) et du dioxyde de carbone (12) sont transformés en monoxyde de carbone et hydrogène, et
- une étape de synthèse (22), lors de laquelle le monoxyde de carbone et l'hydrogène formés lors de l'étape de reformage à sec (21) sont transformés en éther diméthylique (13) et dioxyde de carbone,
**caractérisé en ce que**
l'étape de reformage à sec (21) et l'étape de synthèse (22) sont réalisées à des pressions qui ne diffèrent pas de plus de 3,0 bar, de préférence pas de plus de 1,0 bar, l'une de l'autre.

2. Procédé selon la revendication 1, **caractérisé en ce que** le procédé est réalisé à une pression de 20 bar à 50 bar.

3. Procédé selon la revendication 1 ou 2, **caractérisé en ce qu'**un produit gazeux formé par l'étape de reformage à sec (21) contenant ledit monoxyde de carbone et ledit hydrogène est introduit directement dans l'étape de synthèse (22).

4. Procédé selon l'une quelconque des revendications 1 à 3, **caractérisé en ce que**, lors de l'étape de synthèse (22), le monoxyde de carbone et l'hydrogène formés lors de l'étape de reformage à sec (21) sont transformés en éther diméthylique (13) et dioxyde de carbone jusqu'à un moment à partir duquel l'éther diméthylique est présent en une concentration d'au moins 60 %, 70 %, 80 %, 90 ou 100 % de la concentration à l'équilibre d'éther diméthylique.

5. Procédé selon l'une quelconque des revendications 1 à 4, **caractérisé en ce que** le produit de l'étape de synthèse (22) est séparé en une phase produit (13) contenant principalement de l'éther diméthylique, du méthanol et de l'eau, et un gaz résiduel (14) contenant principalement de l'hydrogène, du monoxyde de carbone, du dioxyde de carbone et du méthane.

6. Procédé selon la revendication 5, **caractérisé en ce que** le gaz résiduel (14) contenant principalement de l'hydrogène, du monoxyde de carbone, du dioxyde de carbone et du méthane est introduit dans l'étape de reformage à sec (21).

7. Procédé selon la revendication 5, **caractérisé en ce que** le gaz résiduel (14) contenant principalement de l'hydrogène, du monoxyde de carbone, du dioxyde de carbone et du méthane est utilisé pour la mise à disposition d'énergie thermique pour l'étape de reformage à sec (21).

8. Procédé selon l'une quelconque des revendications précédentes, **caractérisé en ce que** l'éther diméthylique (13) est mis en réaction lors d'une étape de synthèse d'oléfines pour former un produit comprenant des oléfines, notamment de l'éthylène et/ou du propylène, l'éther diméthylique (13), notamment après séparation uniquement du CO₂, ou la phase produit (13) étant introduit directement dans l'étape de synthèse d'oléfines.
